# EUROPEAN PATENT APPLICATION

(11) **EP 1 767 137 A1**
(43) Date of publication of application: **28.03.2007**
(21) Application number: 05727455.7
(22) Date of filing: 30.03.2005
(51) Int. Cl.: A61B 1/04, A61B 1/00

(54) **MEDICAL INSTRUMENT**

(30) Priority: 18.06.2004 JP 2004181422
(71) Applicant: Olympus Corporation, Tokyo 151-0072 (JP)
(72) Inventor: TAKAYAMA, Masaki c/o Int. Prop. Sup. Dept., Hachioji-shi, Tokyo 192-8512 (JP); HAGIHARA, Masahiro c/o Int. Prop. Sup. Dept., Hachioji-shi, Tokyo 192-8512 (JP); TAKEKOSHI, Satoshi c/o Int. Prop. Sup. Dept., Hachioji-shi, Tokyo 192-8512 (JP); YAMAGUCHI, Takao c/o Int. Prop. Sup. Dept., Hachioji-shi, Tokyo 192-8512 (JP); OMACHI, Kenji c/o Int. Prop. Sup. Dept., Hachioji-shi, Tokyo 192-8512 (JP); SHIMIZU, Masami c/o Int. Prop. Sup. Dept., Hachioji-shi, Tokyo 192-8512 (JP)
(74) Representative: von Hellfeld, Axel
(86) International application number: PCT/JP2005/006157
(87) International publication number: WO 2005/122868

(57) **Abstract**

There is provided medical apparatus in which a member inside a sealed container can be operated securely and whose operability is improved.

An external magnet (58a) provided outside a sealed container (42) is movable with respect to the sealed container (42), and an external member (46) moves the external magnet (58a). An internal magnet (64a) provided in the sealed container (42) is movable with respect to the sealed container (42), and an internal member (60) is moved by movement of the internal magnet (64a). The external magnet (58a) and the internal magnet (64a) are arranged in such a manner that the internal magnet (64a) is moved by the action of a repulsive force between the external magnet (58a) and the internal magnet (64a) based on movement of the external magnet (58a).

## Description

### Technical Field

The present invention relates to medical apparatus which has a sealed container whose inside is sealed and in which various kinds of members in this sealed container is operated by utilizing a magnetic force between magnets arranged outside and inside the sealed container.

### Background Art

As sterilization of medical apparatus used in surgeries, high-pressure steam sterilization (which will be referred to as autoclave sterilization hereinafter) which has a high sterilization performance and is superior in running costs has been conventionally utilized. In this sterilization method, since medical apparatus is exposed to high-pressure steam, various kinds of members are accommodated in an airtight container whose inside is airtightly maintained in order to avoid damage to the medical apparatus. A magnetic force between magnets arranged outside and inside the airtight container is utilized to operate such members isolated from the outside.

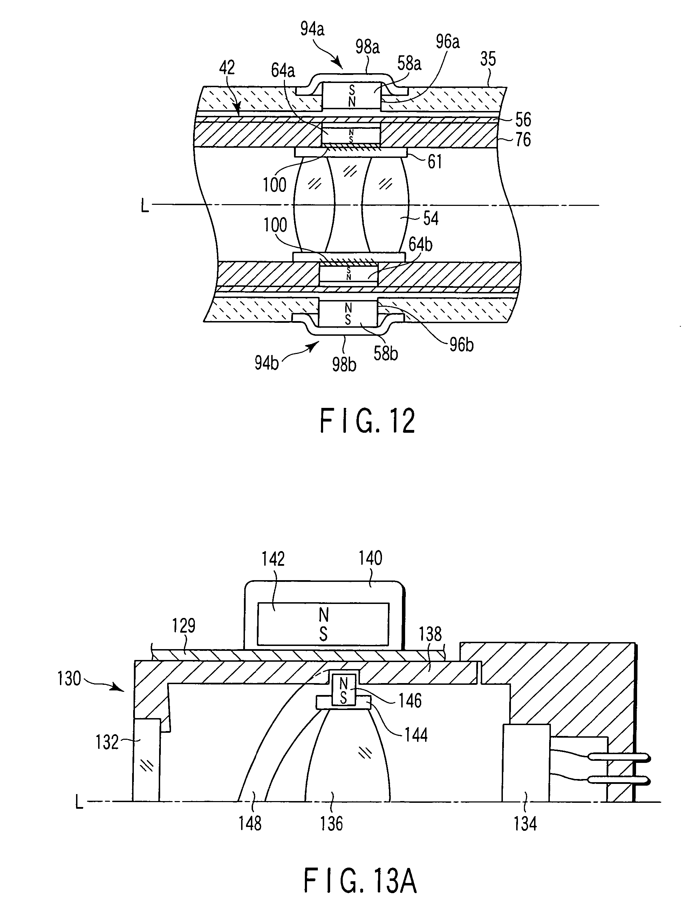

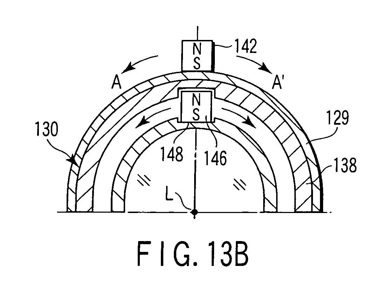

As examples of such medical apparatus, each of U.S.P Nos. 5,359,992, 6,099,467 and 6,522,477 discloses medical apparatus which utilizes an attractive force between magnets. A schematic structure of such medical apparatus will now be described while taking a camera head which picks up an observation image of an endoscope as an example. As shown in FIGS. 13A and 13B, a substantially cylindrical airtight container 130 whose inside is airtightly held is accommodated in a main body portion 129 of this camera head. A cover glass 132 which takes in an observation image of an endoscope is arranged at a distal end of this airtight container 130. On the other hand, an imaging device 134 which picks up an observation image is arranged at a rear end of the airtight container 130. A focusing lens 136 which performs focus adjustment is arranged between the cover glass 132 and the imaging device 134.

A mechanism which performs focus adjustment will now be described. The airtight container 130 has a cylindrical housing 138. A central axis L of this housing 138 matches with an optical axis. An annular focus ring 140 is mounted onto an outer peripheral surface of the housing 38 with the main body portion 129 interposed between them. This focus ring 140 is rotatable around the central axis L with respect to the housing 138. Further, a magnet (which will be referred to as an external magnet 142 hereinafter) is embedded on an inner peripheral surface side of the focus ring 140.

On the other hand, an annular lens frame 144 is mounted outside the focusing lens 136, and this lens frame 144 is mounted onto an inner peripheral surface of the housing 138 to be rotatable around the central axis with respect to the housing 138. A magnet (which will be referred to as an internal magnet 146 hereinafter) is provided to protrude on an outer peripheral surface of the lens frame 144. The external magnet 142 and the internal magnet 146 are arranged in such a manner that different poles face each other with the housing 138 interposed between them, and an attractive force acts between the external magnet 142 and the internal magnet 146. Furthermore, the internal magnet 146 also has a function as a cam pin, and inserted into and engaged with a cam groove 148 formed on the inner peripheral surface of the housing 138. This cam groove 148 is spirally extended on the inner peripheral surface of the housing 138.

When performing focus adjustment, the focus ring 140 is turned to rotate the external magnet 142 around the central axis with respect to the housing 138. Since the attractive force acts between the external magnet 142 and the internal magnet 146, the internal magnet 146 is rotated in accordance with the external magnet 142 (see arrows A and A' in FIG. 13B). Since the internal magnet 146 is rotated along the spiral cam groove 148, the lens frame 144 is rotated around the central axis and moved in the axial direction with respect to the housing 138. That is, when the focus ring 140 is turned, the focusing lens 136 is moved forward and backward along the optical axis. The focus adjustment is carried out in this manner.

### Disclosure of Invention

In the above-described medical apparatus, the internal magnet 146 is moved by utilizing the attractive force between the external magnet 142 and the internal magnet 146, and a distance between the external magnet 142 and the internal magnet 146 is increased when starting an operation of the focus ring 140. Since the magnetic force is in inverse proportion to a square of the distance between the magnets, the magnetic force is suddenly attenuated to lead to slip off when the focus ring 140 should be rapidly moved. In order to avoid this slip off and securely operate members in the airtight container 130, there is adopted a method which performs weighting to avoid rapid movement of the focus ring 140. However, when weighting is carried out, a large control force is required at the time of a turning operation, and operability is deteriorated.

In view of the above-described problems, it is an object of the present invention to provide medical apparatus in which a member inside a sealed container can be operated securely and whose operability is improved.

According to an embodiment of the present invention, medical apparatus is characterized by comprising: a sealed container whose inside is sealed; at least one external magnet which is provided outside the sealed container to be movable with respect to the sealed container; an external member which is provided outside the sealed container and moves the external magnet; at least one internal magnet which is provided in the sealed container to be movable with respect to the sealed container; and an internal member which is provided in the sealed container and moved by movement of the internal magnet, wherein the external magnet and the internal magnet are arranged in such a manner that the internal magnet is moved by the action of a repulsive force between the external magnet and the internal magnet based on movement of the external magnet.

In this embodiment, the external magnet is moved with respect to the sealed container by using the external member, the internal magnet is moved by the action of a repulsive force between the external magnet and the internal magnet, and the internal member is moved by utilizing movement of the internal magnet.

Preferably, the plurality of external magnets is provided, reciprocable with respect to the sealed container and arranged side by side in a reciprocating direction, and the internal magnet is arranged between the external magnets with respect to the reciprocating direction.

In this embodiment, the external magnet arranged on a one-direction side of the internal magnet is moved in the other direction with respect to the sealed container to move the internal magnet in the other direction, and the external magnet arranged on the other-direction side of the internal magnet is moved in the one direction with respect to the sealed container to move the internal magnet in the one direction.

Preferably, the plurality of internal magnets is provided, reciprocable with respect to the sealed container and arranged side by side in a reciprocating direction, and the external magnet is arranged between the internal magnets with respect to the reciprocating direction.

In this embodiment, the internal magnet arranged on a one-direction side of the external magnet is moved in one direction when the external magnet is moved in the one direction with respect to the sealed container, and the internal magnet arranged on the other-direction side of the external magnet is moved in the other direction when the external magnet is moved in the other direction with respect to the sealed container.

Preferably, the sealed container has a cylindrical housing, the external member has an external annular member which is mounted outside the housing to be rotatable around a longitudinal axis of the housing, the internal member has an internal annular member which is mounted inside the housing to be rotatable around the longitudinal axis of the housing, and the external magnet is provided to the external annular member and the internal magnet is provided to the internal annular member.

In this embodiment, the external annular member is operated to rotate the external magnet around the longitudinal axis of the housing with respect to the housing, and to rotate the internal magnet around the longitudinal axis of the housing together with the internal annular member with respect to the housing.

Preferably, the sealed container has a cylindrical housing, the external member has an external annular member which is mounted outside the housing to be movable in a longitudinal axial direction of the housing, the internal member has an internal annular member which is mounted inside the housing to be movable in the longitudinal axial direction of the housing, and the external magnet is provided to the external annular member and the internal magnet is provided to the internal annular member.

In this embodiment, the external annular member is operated to move the external magnet in the longitudinal axial direction of the housing with respect to the housing, and to move the internal magnet in the longitudinal axial direction of the housing together with the internal annular member with respect to the housing.

Preferably, the sealed container has an urging member, and the internal member is movable in one direction by the internal magnet and movable in an opposite direction by urging of the urging member.

In this embodiment, the internal member is moved in one direction by the internal magnet, and the internal member is moved in an opposite direction by urging of the urging member.

Preferably, the sealed container has a cylindrical housing, the external magnet is movable in a longitudinal axial direction of the housing, the internal member has an internal annular member which is mounted inside the housing to be movable in the longitudinal axial direction of the housing, and the internal magnet is provided to the internal annular member.

In this embodiment, the external magnet is moved in one longitudinal axial direction of the housing with respect to the housing to move the internal magnet in the one longitudinal axial direction of the housing together with the internal annular member with respect to the housing, and the internal annular member is moved in the other longitudinal axial direction of the housing with respect to the housing by urging of the urging member.

Preferably, the sealed container has a cylindrical housing, the external magnet is reciprocable in a radial direction of a longitudinal axis of the housing, and the internal magnet is reciprocable in the radial direction.

In this embodiment, the external magnet is moved in a radial direction of the housing with respect to the housing to move the internal magnet in the radial direction with respect to the housing.

According to the present invention, the members in the sealed container can be securely operated, and operability of the medical apparatus is improved.

### Brief Description of Drawings

FIG. 1 is a perspective view showing a schematic structure of an endoscopic system according to a first embodiment of the present invention.
FIG. 2 is a perspective view showing a schematic structure of a camera head according to the first embodiment of the present invention.
FIG. 3A is a longitudinal sectional view showing the camera head according to the first embodiment of the present invention.
FIG. 3B is a transverse sectional view showing the camera head according to the first embodiment of the present invention.
FIG. 4 is a transverse sectional view showing a camera head according to a modification of the first embodiment of the present invention.
FIG. 5 is a transverse sectional view showing a camera head according to a second embodiment of the present invention.
FIG. 6 is a transverse sectional view showing a camera head according to a third embodiment of the present invention.
FIG. 7A is a longitudinal sectional view showing a focus adjustment mechanism of a camera head according to a fourth embodiment of the present invention.
FIG. 7B is a transverse sectional view showing the focus adjustment mechanism of the camera head according to the fourth embodiment of the present invention.
FIG. 7C is a longitudinal sectional view showing an image rotation mechanism of the camera head according to the fourth embodiment of the present invention.
FIG. 8A is a perspective view showing a schematic structure of the camera head according to a fifth embodiment of the present invention.
FIG. 8B is a longitudinal sectional view showing the camera head according to the fifth embodiment of the present invention.
FIG. 8C is a perspective view showing a focus knob according to the fifth embodiment of the present invention.
FIG. 8D is a perspective view showing a sliding ring according to the fifth embodiment of the present invention.
FIG. 9 is a longitudinal sectional view showing a camera head according to a modification of the fifth embodiment of the present invention.
FIG. 10 is a longitudinal sectional view showing a camera head according to a sixth embodiment of the present invention.
FIG. 11A is a transverse sectional view showing a camera head according to a seventh embodiment of the present invention.
FIG. 11B is a perspective view showing hair of an internal magnet according to the seventh embodiment of the present invention.
FIG. 11C is a view illustrating a pressing operation of an external magnet in the camera head according to the seventh embodiment of the present invention.
FIG. 11D is a view illustrating deformation of the hair in the camera head according to the seventh embodiment of the present invention.
FIG. 12 is a longitudinal sectional view showing a camera head according to a modification of the seventh embodiment of the present invention.
FIG. 13A is a longitudinal sectional view showing a conventional camera head.
FIG. 13B is a transverse sectional view showing the conventional camera head.

### Best Mode for Carrying Out the Invention

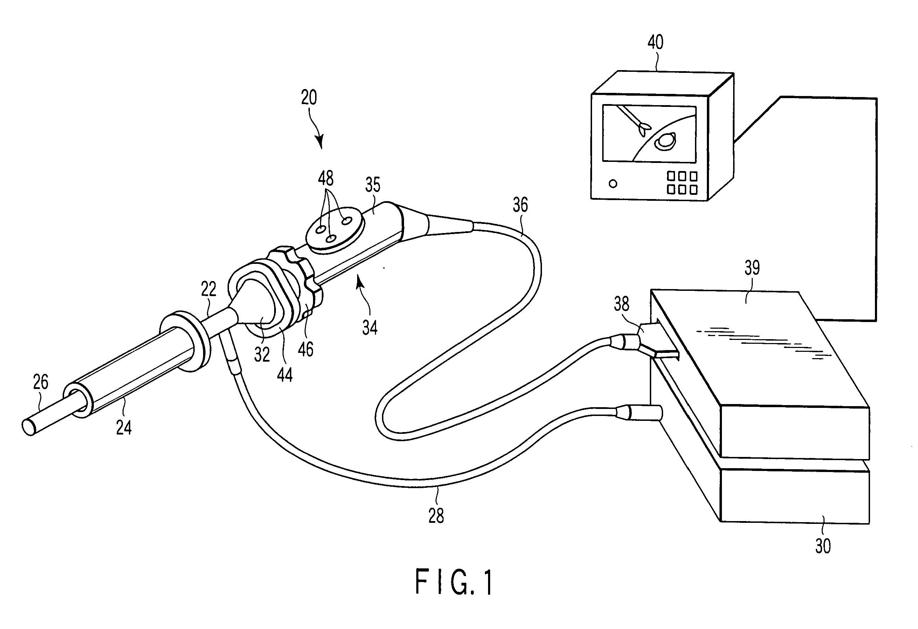

A first embodiment according to the present invention will now be described with reference to FIGS. 1 to 3B. FIG. 1 shows a schematic structure of an endoscopic system 20 according to the first embodiment of the present invention. The endoscopic system 20 according to this embodiment has an endoscope 22 which is used to observe in a body cavity. This endoscope 22 has an inserting portion 26 which is inserted into a body cavity through a sheath 24 or a trocar fixed to an affected part. A light guide 28 which transmits illumination light is extended from a proximal end of this inserting portion 26. An extended end of this light guide 28 is connected with a light source 30. Moreover, an eyepiece portion 32 is continuously provided at the proximal end of the inserting portion 26.

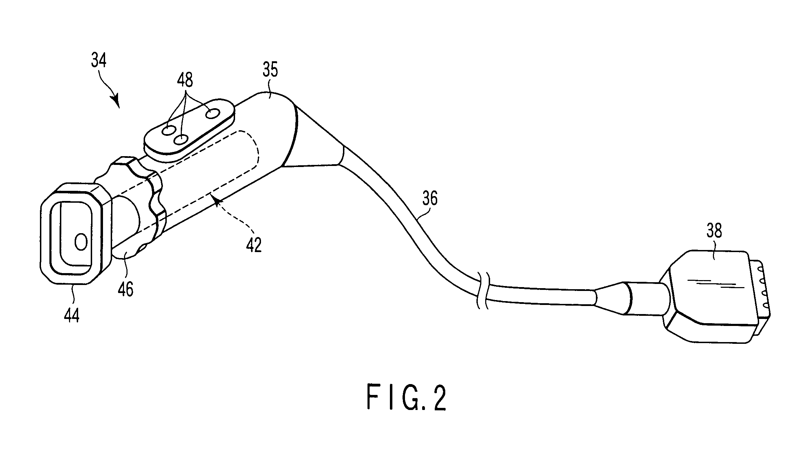

As shown in FIGS. 1 and 2, the endoscope 22 is attached to a camera head 34 which picks up an observation image of the endoscope 22. That is, the eyepiece portion 32 of the endoscope 22 is detachably attached to a connecting portion 44 arranged at a distal end of a main body portion 35 of the camera head 34. Additionally, a focus ring 46 which is used to perform focus adjustment of an observation image of the endoscope 22 is mounted onto an outer peripheral surface of the main body portion 35. Further, a plurality of remote switches 48 is arranged on the outer peripheral surface of the main body portion 35. These remote switches 48 are used for, e.g., white balance or start/stop of video recording.

Furthermore, a camera cord 36 which transmits a picture signal of a pick up observation image is extended from a rear end of the main body portion 35. A camera plug 38 is arranged at an extended end of this camera cord 36, and this camera plug 38 is connected with an image processing device 39. This image processing device 39 is connected with a TV monitor 40 and displays an observation image in this TV monitor 40.

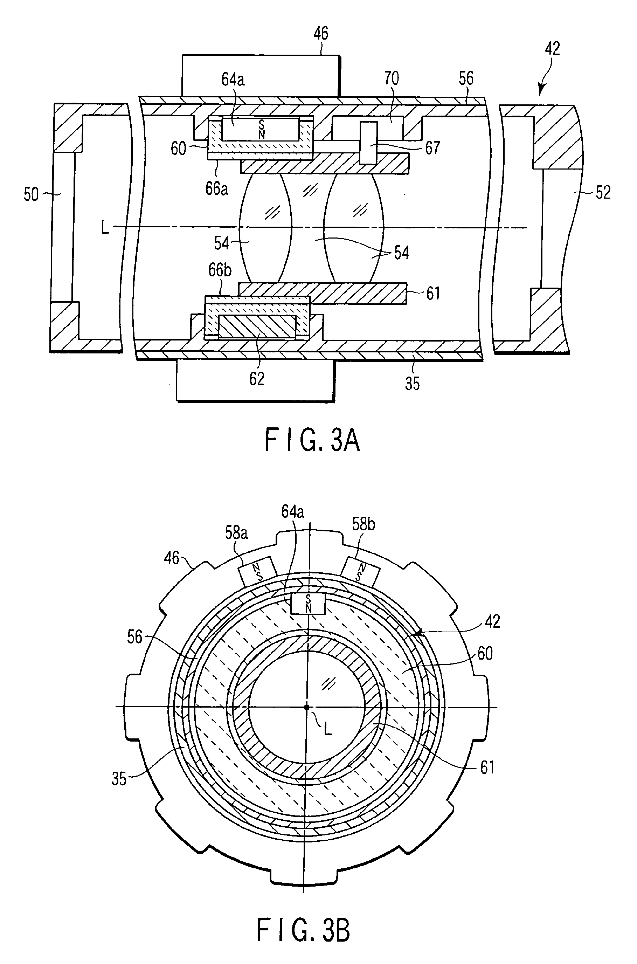

As shown in FIG. 2, an airtight container 42 as a sealed container is accommodated in the main body portion 35 of the camera head 34. The inside of this airtight container 42 can be airtightly held with respect to, e.g., high-temperature and high-pressure steam used for autoclave sterilization. As shown in FIG. 3A, a cover glass 50 which takes an observation image of the endoscope 22 (see FIG. 1) is arranged at a distal end of the airtight container 42. On the other hand, an imaging device which picks up an observation image, e.g., a CCD 52 is arranged at a rear end of the airtight container 42. A focusing lens group 54 which is used to perform focus adjustment is arranged between the cover glass 50 and the CCD 52.

A mechanism which performs focus adjustment will now be described with reference to FIGS. 3A and 3B. The airtight container 42 has a cylindrical housing 56, and a central axis L (a longitudinal axis) of the housing 56 matches with an optical axis. The annular focus ring 46 as an external member (an external annular member) is mounted onto an outer peripheral surface of this housing 56 with the main body portion 35 interposed between them. This focus ring 46 is rotatable around the central axis L of the housing 56 with respect to the housing 56. Irregularities are formed on an outer peripheral surface of the focus ring 46 to facilitate a turning operation.

Two magnets are embedded on an inner peripheral surface side of the focus ring 46. These magnets are arranged outside the airtight container 42, and they will be referred to as first and second external magnets 58a and 58b hereinafter. These first and second external magnets 58a and 58b are arranged to be separated from each other by a predetermined distance in a peripheral direction. Moreover, the first and second external magnets 58a and 58b are arranged in such a manner that north poles are provided on an outer side in a radial direction of the central axis L and south poles are provided on an inner side in the radial direction of the same. A magnetic force of the first external magnet 58a is substantially equal to that of the second external magnet 58b.

A guide groove 62 is formed on an inner peripheral surface of the housing 56 over an entire periphery. An annular drive ring 60 as an internal member (an internal annular member) is fitted in this guide groove 62. Additionally, the drive ring 60 is rotatable around the central axis with respect to the housing 56, and its movement in the axial direction of the central axis L of the drive ring 60 is restricted.

One magnet is embedded on an outer peripheral surface side of the drive ring 60. This magnet is arranged in the airtight container 42, and it will be referred to as an internal magnet 64a hereinafter. This internal magnet 64a is arranged between the first external magnet 58a and the second external magnet 58b with respect to a peripheral direction and aligned with the first and second external magnets 58a and 58b with respect to the axial direction. Further, the internal magnet 64a is arranged in such a manner that its south pole is provided on the outer side in the radial direction of the central axis L of the housing 56 and its north pole is provided on the inner side in the radial direction of the same.

A repulsive force acts between the first and second external magnets 58a and 58b and the internal magnet 64a. Further, the drive ring 60 is usually positioned in such a manner that a repulsive force between the first external magnet 58a and the internal magnet 64a becomes substantially equal to a repulsive force between the second external magnet 58b and the internal magnet 64a, i.e., a distance between the first external magnet 58a and the internal magnet 64 becomes substantially equal to a distance between the second external magnet 58b and the internal magnet 64a. An arrangement of magnets consisting of the first and second external magnets 58a and 58b and the internal magnet 64a mentioned above will be referred to as a basic arrangement.

A female screw type first feed screw 66a is formed on an inner peripheral surface of the drive ring 60. Furthermore, a distal end side of a cylindrical lens frame 61 is fitted into an inner cavity of the drive ring 60, and a second feed screw 66b formed on a distal end side of the lens frame 61 is screwed to the first feed screw 66a of the drive ring 60. Moreover, the focusing lens group 54 is fixed on an inner peripheral surface of the lens frame 61.

On the other hand, a cam pin 67 is provided to protrude on an outer peripheral surface of the lens frame 61 on a rear end side. This cam pin 67 is inserted into and engaged with a cam groove 70 formed on the inner peripheral surface of the housing 56. This cam groove 70 is extended in the axial direction. That is, the lens frame 61 is restricted from rotating around the central axis by the cam pin 67 and the cam groove 70 but movable in the axial direction alone.

A function of the endoscopic system 20 according to this embodiment will now be described. When using the endoscopic system 20, autoclave sterilization is performed prior to use. Since the inside of the airtight container 42 is airtightly held, steam rarely enters, and the focusing lens group 54, the CCD 52 and others in the airtight container 42 are prevented from misting over. After autoclave sterilization, the endoscope 22 is attached to the camera head 34. Focus adjustment must be carried out in accordance with the endoscope 22 in order to obtain an appropriate observation image.

When effecting focus adjustment, the focus ring 46 is turned around a central axis with respect to the housing 56. As a result, the first and second external magnets 58a and 58b are also rotated around the central axis. When the focus ring 46 is turned in a direction extending from the first external magnet 58a toward the second external magnet 58b, a distance between the first external magnet 58a and the internal magnet 64a becomes smaller than a distance between the second external magnet 58b and the internal magnet 64a. Since a magnetic force is in inverse proportion to a square of the distance between the magnets, a larger repulsive force than that between the second external magnet 58b and the internal magnet 64a acts between the first external magnet 58a and the internal magnet 64a. As a result, the internal magnet 64a is rotated together with the drive ring 60 in the direction extending from the first external magnet 58a toward the second external magnet 58b.

When the drive ring 60 is turned, the first feed screw 66a is rotated around the central axis. The lens frame 61 is restricted from rotating around the central axis by the cam pin 67 and the cam groove 70, and the lens frame 61 is moved in one axial direction by a mutual function of the first and second feed screws 66a and 66b. Since the central axis L of the housing 56 matches with the optical axis, the focusing lens group 54 is moved in one optical axial direction.

On the other hand, when the focus ring 46 is turned in a direction extending from the second external magnet 58b toward the first external magnet 58a, the repulsive force between the second external magnet 58b and the internal magnet 64a is increased, and the focusing lens group 54 is moved in the other optical axis direction. That is, when the focus ring 46 is turned, the focusing lens group 54 is moved forward or backward along the optical axis. Focus adjustment of the focus ring 46 is carried out in this manner. Upon completion of focus adjustment, observation using the endoscope 22 is effected.

Therefore, the endoscopic system 20 according to this embodiment demonstrates the following effects. The external magnets 58a and 58b are moved with respect to the airtight container 42 by turning the focus ring 46, and the internal magnet 64a is moved together with the drive ring 60 by utilizing the action of the repulsive force between the external magnet 58a or 58b and the internal magnet 64a. In the embodiment, the repulsive force between the external magnet 58a or 58b and the internal magnet 64a is utilized, and the distance between the external magnet 58a or 58b and the internal magnet 64a is reduced at the time of start of an operation of the focus ring 46. Since the magnetic force is in inverse proportion to a square of the distance between magnets, the magnetic force is suddenly increased even if the focus ring 46 is rapidly moved, and hence slip off rarely occurs. Therefore, the focusing lens group 54 can be securely operated in accordance with the drive ring 60. Additionally, weighting of the focus ring 46 does not have to be performed in order to avoid rapid rotation of the focus ring 46. Therefore, a large control force is not required when turning the focus ring 46, thereby improving operability.

Further, the first and second external magnets 58a and 58b can reciprocate in a peripheral direction with respect to the airtight container 42 and are arranged side by side in the peripheral direction. Furthermore, the internal magnet 64a is arranged between the first external magnet 58a and the second external magnet 58b in the peripheral direction. Therefore, when the focus ring 46 is turned in the direction extending from the first external magnet 58a toward the second external magnet 58b, the internal magnet 64a is rotated together with the drive ring 60 in the direction extending from the first external magnet 58a toward the second external magnet 58b by the first external magnet 58a. On the other hand, when the focus ring 46 is turned in an opposite direction, the drive ring 60 is rotated in the opposite direction. That is, the drive ring 60 is capable of reciprocating. When the first and second external magnets 58a and 58b and the internal magnet 64a are arranged in this manner like this embodiment, the internal member such as a drive ring 60 can perform a reciprocating motion, and this embodiment can be applied to various mechanisms requiring the reciprocating motion.

Although the focusing lens group 54 is moved forward and backward along the optical axis to perform focus adjustment in this embodiment, focus adjustment is likewise possible when the CCD 52 is arranged in place of the focusing lens group 54 and the CCD 52 is moved forward and backward along the optical axis. Furthermore, in this embodiment, a combination of the focus ring 46 and the focusing lens group 54 is used to effect focus adjustment. Alternatively, a combination of a zoom ring and a variable-power lens group may be used in order to carry out variable-power adjustment of an observation image. Moreover, both focus adjustment and variable-power adjustment may be used.

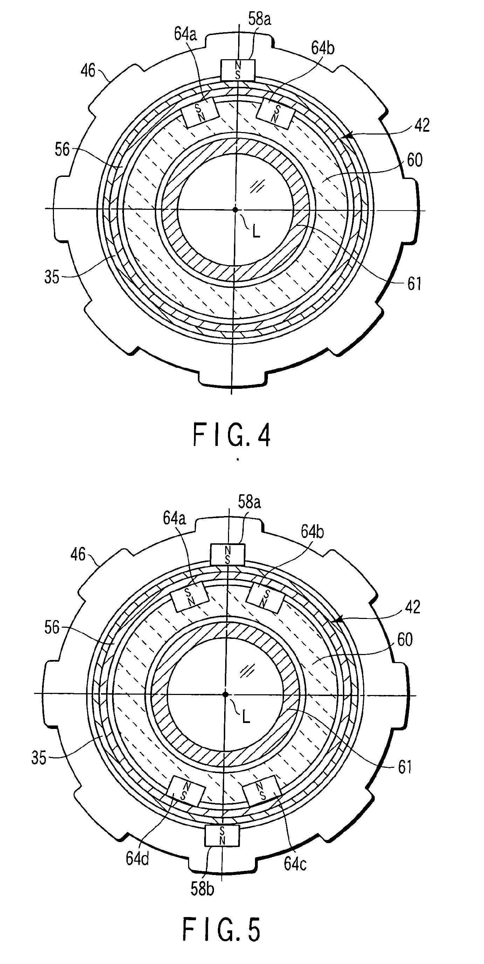

FIG. 4 shows a modification of the first embodiment according to the present invention. Like reference numerals denote structures having the same functions as those of the first embodiment, thereby obviating a description thereof. This modification uses one external magnet and two internal magnets as a basic arrangement. That is, one external magnet 58a is embedded on an inner peripheral surface side of a focus ring 46 according to this modification. On the other hand, first and second internal magnets 64a and 64b are embedded on an outer peripheral surface side of a drive ring 60. These first and second internal magnets 64a and 64b are arranged to be separated from each other by a predetermined distance in a peripheral direction. Additionally, the external magnet 58a is arranged between the first internal magnet 64a and the second internal magnet 64b with respect to a peripheral direction. A magnetic force intensity of the first internal magnet 64a is substantially equal to that of the second internal magnet 64b, and the drive ring 60 is usually positioned in such a manner that a distance between the external magnet 58a and the first internal magnet 64a becomes substantially equal to a distance between the external magnet 58a and the second internal magnet 64b.

A function and an effect of an endoscopic system 20 according to this modification will now be described. When the focus ring 46 is turned in a direction extending from the second internal magnet 64b toward the first internal magnet 64a, the distance between the external magnet 58a and the first internal magnet 64a becomes smaller than the distance between the external magnet 58a and the second internal magnet 64b. Therefore, a larger repulsive force than that between the external magnet 58a and the second internal magnet 64b acts between the external magnet 58a and the first internal magnet 64a. As a result, the first internal magnet 64a is rotated together with the drive ring 60 in a direction extending from the second internal magnet 64b toward the first internal magnet 64a. On the other hand, when the focus ring 46 is turned in an opposite direction, the drive ring 60 is rotated in the opposite direction. Therefore, the endoscopic system 20 according to this modification demonstrates the same effect as that of the endoscopic system 20 according to the first embodiment.

FIG. 5 shows a second embodiment according to the present invention. Like reference numerals denote structures having the same functions as those of the modification of the first embodiment, thereby obviating a description thereof. This embodiment uses the two basic arrangements equal to that in the modification of the first embodiment. That is, a first external magnet 58a and first and second internal magnets 64a and 64b are arranged like the modification of the first embodiment. Further, the second external magnet 58b is arranged in a focus ring 46 to be symmetrical to the first external magnet 58a with respect to a central axis L. Furthermore, a pair of third and fourth internal magnets 64c and 64d is arranged in a drive ring 60 to be symmetrical to a pair of first and second internal magnets 64a and 64b with respect to the central axis L. That is, the second external magnet 58b and the third and fourth internal magnets 64c and 64d are arranged like the first external magnet 58a and the first and second internal magnets 64a and 64b, thereby forming the basic arrangements.

A function and an effect of an endoscopic system 20 according to this embodiment will now be described. When the focus ring 46 is turned, the drive ring 60 is rotated by the action of a repulsive force between the first external magnet 58a and the first and second internal magnets 64a and 64b as well as a repulsive force between the second external magnet 58b and the third and fourth internal magnets 64c and 64c. Since the two basic arrangements are used in this embodiment as described above, the drive ring 60 can be rotated by utilizing a stronger repulsive force than that in a case where one basic arrangement is adopted. Therefore, follow-up properties of the drive ring 60 with respect to the focus ring 46 are improved.

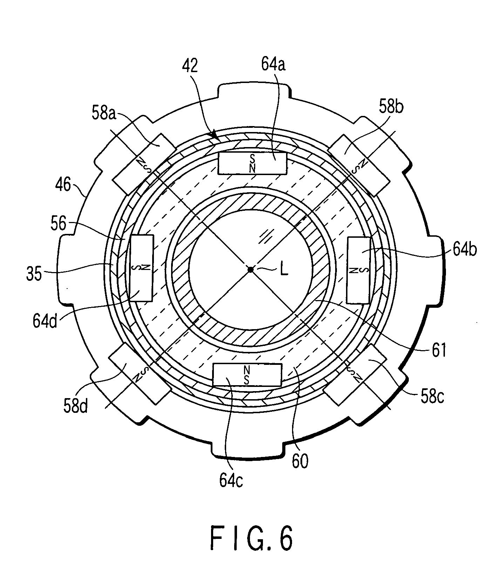

FIG. 6 shows a third embodiment according to the present invention. Like reference numerals denote structures having the same functions as those of the first embodiment, thereby obviating a description thereof. In this embodiment, external magnets and internal magnets are alternately arranged over an entire periphery. That is, first to fourth external magnets 58a, 58b, 58d and 58d are arranged in a focus ring 46, and first to fourth internal magnets 64a, 64b, 64c and 64d are arranged in a drive ring 60. Moreover, the first to fourth external magnets 58a, ..., 58d and the first to fourth internal magnets 64a, ..., 64d are alternately arranged in sequence over the entire periphery.

Therefore, the first and second external magnets 58a and 58b and the first internal magnet 64a form the same basic arrangement as that in the first embodiment. Likewise, the second and third external magnets 58b and 58c and the second internal magnet 64b or the like form the same basic arrangement as that in the first embodiment. Further, it can be said that the second external magnet 58b and the first and second internal magnets 64a and 64b or the like form the same basic arrangement as that in the modification of the first embodiment.

A function and an effect of an endoscopic system 20 according to this embodiment will now be described. When the focus ring 46 is turned in one peripheral direction, the drive lens is rotated by the action of a repulsive force between the first external magnet 58a and the first internal magnet 64a, between the second external magnet 58b and the second internal magnet 64b and others. On the other hand, when the focus ring 46 is turned in an opposite direction, the drive lens is rotated by the action of a repulsive force between the first external magnet 58a and the fourth internal magnet 64d, between the second external magnet 58b and the first internal magnet 64a or the like. As described above, the four basic arrangements are used in this embodiment, and follow-up properties of the drive ring 60 with respect to the focus ring 46 are further improved. Furthermore, the internal magnet 64a and the external magnet 58a are alternately arranged in sequence over the entire periphery. That is, a minimum number of magnets required to form a provided number of basic arrangements are used. Therefore, a size of a camera head 34 can be reduced.

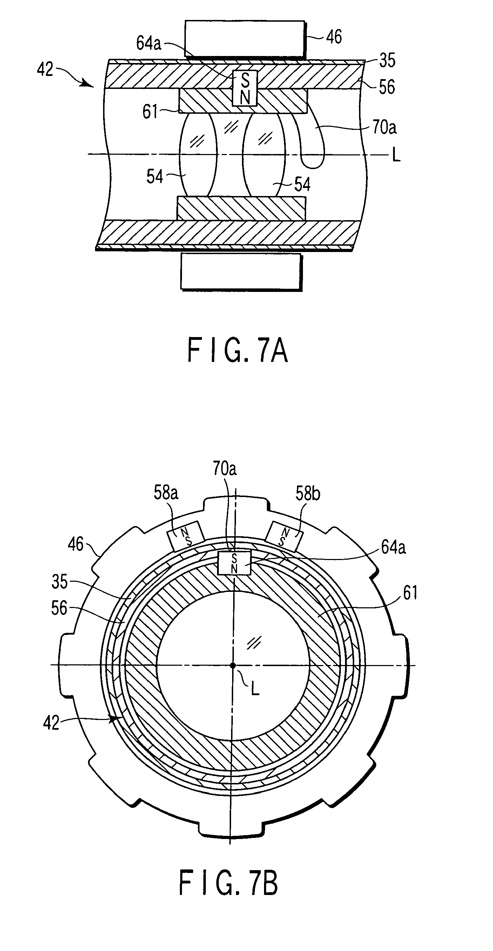

FIGS. 7A to 7C show a fourth embodiment according to the present invention. Like reference numerals denote structures having the same functions as those of the first embodiment, thereby obviating a description thereof. As shown in FIGS. 7A and 7B, a cylindrical lens frame 61 as an internal member (an internal annular member) are mounted onto an inner peripheral surface of a housing 56 to be rotatable around a central axis L of the housing 56. A first internal magnet 64a is provided to protrude on an outer peripheral surface of this lens frame 61. This first internal magnet 64a has a function as a cam pin, and is inserted into and engaged with a first cam groove 70a formed on an inner peripheral surface of the housing 56. This first cam groove 70a is spirally extended on the inner peripheral surface of the housing 56. First and second external magnets 58a and 58b of the focus ring 46 extend over the axial range in which the spiral first cam groove 70a is extended, namely, the first internal magnet 64a can move. As shown in FIG. 7B, the arrangement of the first and second external magnets 58a and 58b and the first internal magnet 64a with respect to a peripheral direction is the same as that in the first embodiment.

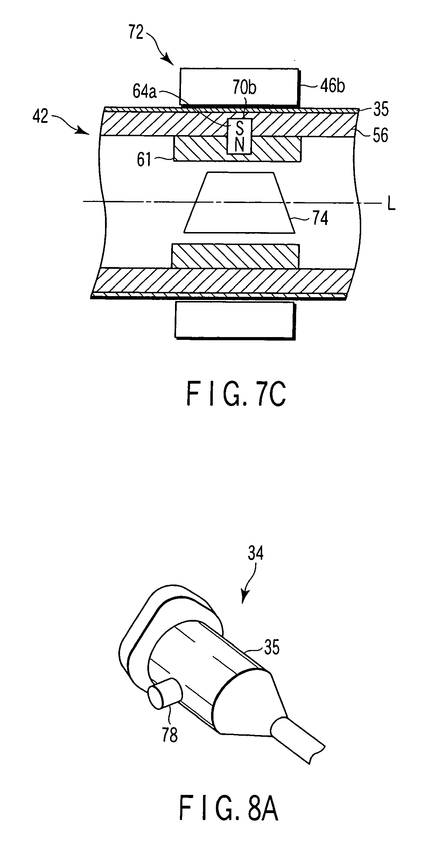

Moreover, a camera head according to this embodiment has an image rotator 72 shown in FIG. 7C. This image rotator 72 has a rotation ring 46b, non-illustrated third and fourth external magnets, a second internal magnet 64b, a second cam groove 70b and a prism frame 61b having the same structure respectively as that of a lens frame 61, the focus ring 46, the first and second external magnets 58a and 58b, the first internal magnet 64a and the first cam groove 70a which are depicted in FIGS. 7A and 7B. However, the second cam groove 70b is extended in a peripheral direction of the central axis L. Additionally, a trapezoidal prism 74 which rotates an observation image around an optical axis is arranged in an inner cavity of the prism frame 61b.

A function and an effect of an endoscopic system 20 according to this embodiment will now be described. When performing focus adjustment, the focus ring 46 is turned. As a result, like the first embodiment, the first internal magnet 64a is rotated around the central axis. At this time, the first internal magnet 64a is slid along the spiral first cam groove 70a, and the lens frame 61 is rotated around the central axis and moved in the axial direction. The focus adjustment is carried out in this manner. Further, when rotation of an observation image is desired, the rotation ring 46b is turned. As a result, the second internal magnet 64b is rotated around the central axis like the first internal magnet 64a, the second internal magnet 64b is slid along the second cam groove 70b, and the prism frame 61b is rotated around the central axis. Furthermore, the trapezoidal prism 74 is rotated around the optical axis, thereby rotating the observation image. In this embodiment, components such as a drive ring 60 are not used in order to convert a rotary motion of the first internal magnet 64a into a linear motion, and hence the number of components is reduced.

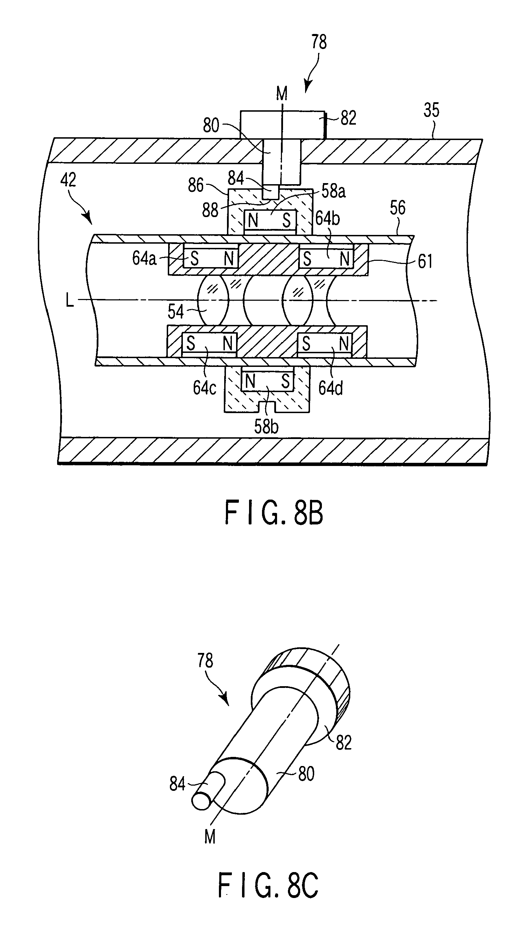

FIGS. 8A to 8D show a fifth embodiment according to the present invention. Like reference numerals denote structures having the same functions as those of the first embodiment, thereby obviating a description thereof. As shown in FIG. 8A, a focus knob 78 required to perform focus adjustment is arranged on a main body portion 35 of a camera head 34 according to this embodiment. As shown in FIGS. 8B and 8C, this focus knob 78 has a cylindrical column portion 80 which pierces the main body portion 35. This cylindrical column portion 80 is rotatable around its own central axis M with respect to the main body portion 35. A flange-shaped expanded diameter portion 82 which is turned is arranged at an outer end of the cylindrical column portion 80. On the other hand, a protruding portion 84 is extended on an inner end surface of the cylindrical column portion 80 in the axial direction of the central axis M. This protruding portion 84 is arranged to be eccentric with respect to the central axis M of the cylindrical column portion 80.

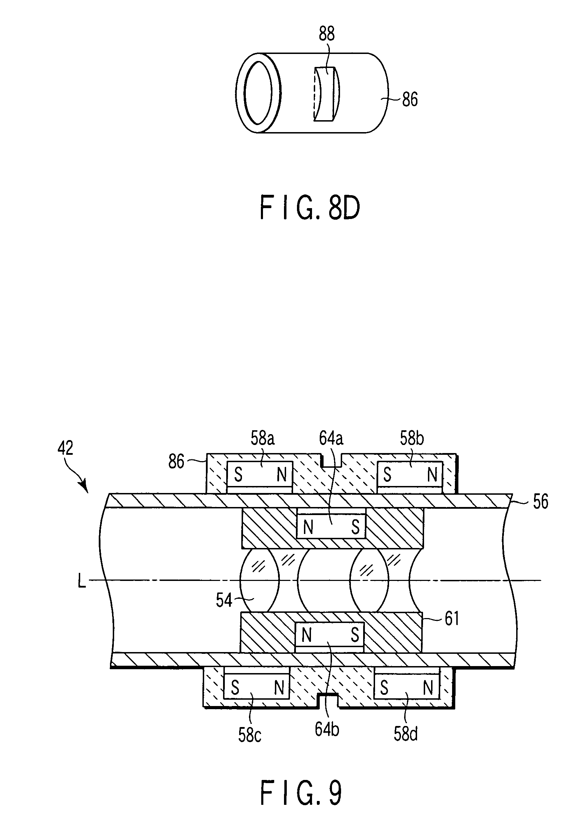

As shown in FIGS. 8B and 8D, this protruding portion 84 is inserted into and engaged with a notch portion 88 formed in an outer peripheral surface of a cylindrical sliding ring 86 as an external member (an external annular member). This notch portion 88 is extended to be perpendicular to both a radial direction and the axial direction. Furthermore, the sliding ring 86 is mounted outside the housing 56 to be slidable with respect to the housing 56 in an axial direction of a central axis L of the housing 56. A first external magnet 58a is embedded on an inner peripheral surface side of the sliding ring 86. The first external magnet 58a is arranged in such a manner that a north pole is provided on a distal end side (a left side in the drawing) in the axial direction and a south pole is provided on a rear end side.

A cylindrical lens frame 61 is mounted onto the inner peripheral surface of the housing 56 to be slidable in the axial direction. First and second internal magnets 64a and 64b are embedded on an outer peripheral surface side of the lens frame 61. These first and second internal magnets 64a and 64b are arranged to be separated from each other by a distance in the axial direction, and the first internal magnet 64a is arranged on the distal end side whilst the second internal magnet 64b is arranged on the rear end side. Moreover, the first and second internal magnets 64a and 64b are arranged in such a manner that south poles are provided on the distal end side in the axial direction and north poles are provided on the rear end side.

Additionally, the first external magnet 58a is arranged between the first internal magnet 64a and the second internal magnet 64b in the axial direction, and aligned with the first and second internal magnets 64a and 64b with respect to a peripheral direction. A magnetic force intensity of the first internal magnet 64a is substantially equal to that of the second internal magnet 64b, and the lens frame 61 is usually positioned with such an arrangement as a distance between the first external magnet 58a and the first internal magnet 64a becomes substantially equal to a distance between the first external magnet 58a and the second internal magnet 64b. Here, the first external magnet 58a and the first and second internal magnets 64a and 64b form a basic arrangement.

Further, a second external magnet 58b is arranged in the sliding ring 86 to be symmetrical to the first external magnet 58a with respect to the central axis L. Furthermore, third and fourth internal magnets 64c and 64d are arranged in the lens frame 61 to be symmetrical to the first and second internal magnets 64a and 64b with respect to the central axis L. That is, the second external magnet 58b and the third and fourth internal magnets 64c and 64d form a basic structure.

A function of an endoscopic system 20 according to this embodiment will now be described. When performing focus adjustment, the focus knob 78 is turned. When the focus knob 78 is turned in one direction, the protruding portion 84 of the focus knob 78 is rotated in one direction around the central axis M of the cylindrical column portion 80. As a result, the protruding portion 84 is slid along the notch portion 88 of the sliding ring 86, and the sliding ring 86 is slid in one axial direction of the central axis L of the housing 56.

Here, it is assumed that the sliding ring 86 is moved in a direction extending from the second internal magnet 64b toward the first internal magnet 64a, namely, it is moved to the distal end side. In this case, a distance between the first external magnet 58a and the first internal magnet 64a is reduced to be smaller than a distance between the first external magnet 58a and the second internal magnet 64b. Further, a larger repulsive force than that between the first external magnet 58a and the second internal magnet 64b acts between the first external magnet 58a and the first internal magnet 64a. As a result, the first internal magnet 64a is moved toward the distal end side together with the lens frame 61. The second external magnet 58b and the third and fourth internal magnets 64c and 64d are likewise operated.

On the other hand, when the focus knob 78 is rotated in an opposite direction, the lens frame 61 is moved to the rear end side. That is, when the focus knob 78 is turned, the focusing lens group 54 is moved forward or backward along the optical axis.

Therefore, the endoscopic system 20 according to this embodiment demonstrates the following effect. A rotary motion of the focus knob 78 is converted into a linear motion of the sliding ring 86, and the linear motion of the sliding ring 86 is converted into a linear motion of the lens frame 61 by the first and second external magnets 58a and 58b and the first to fourth internal magnets 64a, ..., 64d. That is, as different from the first embodiment, a mechanism which converts a rotary motion into a linear motion is arranged outside the airtight container 42, and maintenance of this mechanism is facilitated.

It is to be noted that the basic arrangements are symmetrically provided with respect to the central axis L in this embodiment, the three or more arrangements may be arranged around the central axis. In this case, the lens frame 61 can be slid by using a stronger repulsive force than that in the case where the two basic arrangements are used, thereby improving follow-up properties of the lens frame 61 with respect to the sliding ring 86.

FIG. 9 shows a modification of the fifth embodiment according to the present invention. Like reference numerals denote structures having the same functions as those of the fifth embodiment, thereby obviating a description thereof. This modification uses two external magnets and one internal magnet as a basic arrangement.

On an inner peripheral surface side of a sliding ring 86, first and second external magnets 58a and 58b are arranged to be separated from each other by an amount in an axial direction. Further, on an outer peripheral surface side of a lens frame 61, a first internal magnet 64a is arranged between the first external magnet 58a and the second external magnet 58b with respect to the axial direction. Furthermore, third and fourth external magnets 58c and 58d and a second internal magnet 64b are arranged to be symmetrical to the first and second external magnets 58a and 58b and the first internal magnet 64a with respect to a central axis L.

A function and an effect of an endoscopic system 20 according to this embodiment will now be described. When the sliding ring 86 is moved in a direction extending from the second external magnet 58b toward the first external magnet 58a, a larger repulsive force than that between the first external magnet 58a and the first internal magnet 64a acts between the second external magnet 58b and the first internal magnet 64a. As a result, the first internal magnet 64a is moved together with the lens frame 61 in the direction extending from the second external magnet 58b to the first external magnet 58a. On the other hand, when the sliding ring 86 is moved in an opposite direction, the lens frame 61 is moved in an opposite direction. Therefore, the endoscopic system 20 according to this modification demonstrates the same effect as that of the endoscopic system 20 according to the fifth embodiment.

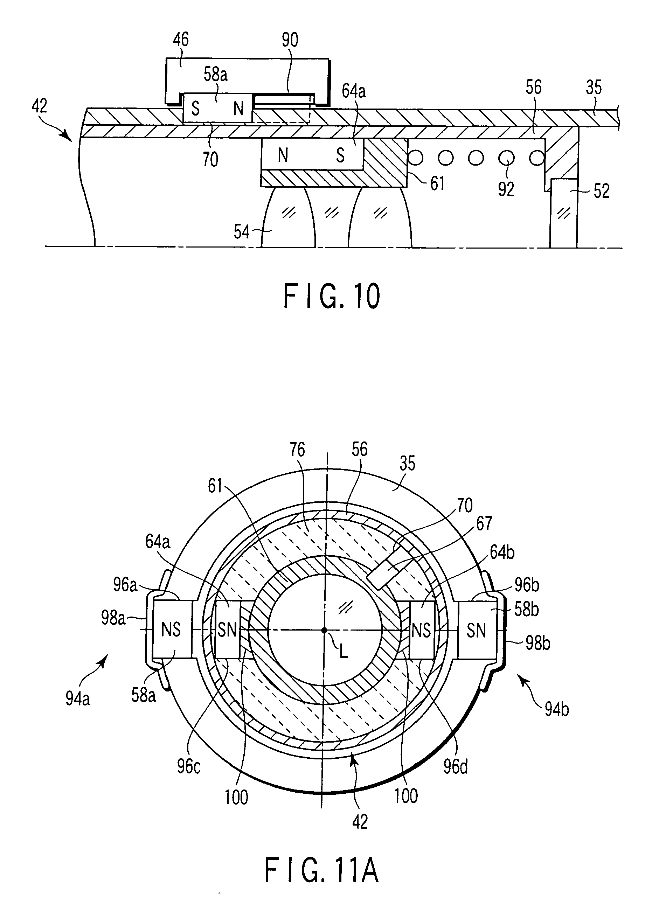

FIG. 10 shows a sixth embodiment according to the present invention. Like reference numerals denote structures having the same functions as those of the first embodiment, thereby obviating a description thereof. A slide groove 90 is extended in an axial direction of a central axis L of a housing 56 on an inner peripheral surface of a focus ring 46 according to this embodiment. A part of an external magnet 58a provided on an outer side in a radial direction of the central axis L is accommodated in this slide groove 90 to be slidable in the axial direction. This external magnet 58a is arranged in such a manner that its south pole is provided on a distal end side in the axial direction and its north pole is provided on a rear end side. Furthermore, a part of the external magnet 58a on an inner side in the radial direction is slidably accommodated in a cam groove 70 formed on an outer peripheral surface of a main body portion 35. This cam groove 70 is spirally extended with respect to the central axis L.

A cylindrical lens frame 61 is mounted inside an inner peripheral surface of the housing 56 to be slidable in the axial direction. This lens frame 61 is arranged on a rear end side of the external magnet 58a with respect to the axial direction. An internal magnet 64a is embedded on an outer peripheral surface side of the lens frame 61. This internal magnet 64a is arranged in such a manner that its north pole is provided on a distal end side with respect to the axial direction and its south pole is provided on the rear end side. Moreover, the internal magnet 64a extends in an entire peripheral range in which the spiral cam groove 70 is extended, i.e., the external magnet 58a can move. Alternatively, the plurality of internal magnets 64a may be arranged over the entire periphery. The lens frame 61 is urged toward the rear end side by a repulsive force acting between the external magnet 58a and the internal magnet 64a. On the other hand, an elastic member as an urging member, e.g., a spring 92 is compressed and arranged between the rear end side of the lens frame 61 and a rear end wall of the housing 56. A spring force of this spring 92 urges the lens frame 61 toward the distal end side. The lens frame 61 is usually arranged at a position where the repulsive force between the external magnet 58a and the internal magnet 64a is balanced by the spring force of the spring 92.

A function of an endoscopic system 20 according to this embodiment will now be described. When performing focus adjustment, the focus ring 46 is turned. When the focus ring 46 is turned around the central axis, the external magnet 58a is slid along the spiral cam groove 70 and moved in the axial direction along the slide groove 90 of the focus ring 46. Here, when the external magnet 58a is moved toward the rear end side, a distance between the external magnet 58a and the internal magnet 64a is reduced, and the repulsive force between the external magnet 58a and the internal magnet 64a is increased. As a result, the internal magnet 64a is moved together with the lens frame 61 toward the rear end side. Additionally, the lens frame 61 is stopped at the position where the repulsive force between the external magnet 58a and the internal magnet 64a is balanced by the spring force.

On the other hand, when the focus ring 46 is turned in an opposite direction, the external magnet 58a is moved toward the distal end side. In this case, the distance between the external magnet 58a and the internal magnet 64a is increased, and the repulsive force between the external magnet 58a and the internal magnet 64a is reduced. As a result, the lens frame 61 is moved toward the distal end side by the spring force toward the distal end side which is applied to the lens frame 61. When the lens frame 61 is moved toward the distal end side, the distance between the external magnet 58a and the internal magnet 64a is reduced, and the repulsive force between the external magnet 58a and the internal magnet 64a is increased. Further, the lens frame 61 is stopped at the position where the repulsive force between the external magnet 58a and the internal magnet 64a is balanced by the spring force.

Therefore, the endoscopic system 20 according to this embodiment demonstrates the following effect. The repulsive force between the internal magnet 64a and the external magnet 58a is utilized to move the lens frame 61 in one direction, and the spring force of the spring 92 is utilized to move the lens frame 61 in the opposite direction. That is, since one-way movement in a reciprocating motion is realized by the spring 92, the number of magnets can be reduced.

FIGS. 11A to 11D show a seventh embodiment according to the present invention. Like reference numerals denote structures having the same functions as those of the first embodiment, thereby obviating a description thereof. As shown in FIG. 11A, in a main body portion 35 according to this embodiment, first and second switches 94a and 94b are arranged at positions facing each other with respect to a central axis L of a housing 56.

These first and second switches 94a and 94b have first and second through holes 96a and 96b which are bored in a radial direction of the central axis L of the main body portion 35. First and second tabular members 98a and 98b which cover these first and second through holes 96a and 96b and have flexibility are respectively provided on an outer peripheral surface of the main body portion 35. Each of these first and second tabular members 98a and 98b has a convex shape in which a part covering the first or second through hole 96a or 96b protrudes toward the outside in the radial direction. Further, first and second external magnets 58a and 58b are respectively accommodated in the first and second through holes 96a and 96b to be slidable in the radial direction. These first and second external magnets 58a and 58b are arranged in such a manner that north poles are provided on the outer side with respect to the radial direction and south poles are provided on the inner side with respect to the radial direction. Radial outer surfaces of the first and second external magnets 58a and 58b are fixed on the radial inner surfaces of the convex parts of the first and second tabular members 98a and 98b.

That is, the radial outer parts of the first and second external magnets 58a and 58b protrude from the main body portion 35 and can be pressed. In this embodiment, the first and second tabular members 98a and 98b and the main body portion 35 form an external member.

A cylindrical inner main body portion 76 is mounted in a housing 56. Third and fourth through holes 96c and 96d are formed in this inner main body portion 76 in the radial direction. These third and fourth through holes 96c and 96d are formed at symmetrical positions with respect to the central axis L and respectively aligned with the first and second through holes 96a and 96b with respect to a peripheral direction.

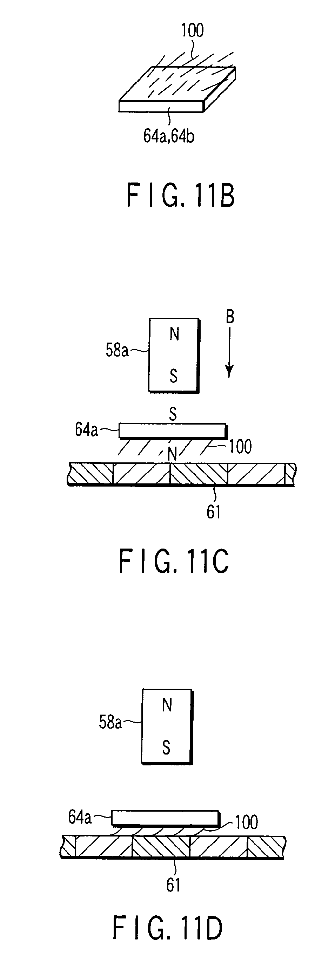

First and second internal magnets 64a and 64b are accommodated in the third and fourth through holes 96c and 96d to be slidable in the radial direction. These first and second internal magnets 64a and 64b are arranged in such a manner that south poles are provided on the outer side with respect to the radial direction and north poles are provided on the inner side with respect the radial direction. Hair 100 is planted on the inner surfaces of the first and second internal magnets 64a and 64b in the radial direction to be inclined in one direction within a plane perpendicular to the central axis L (see FIG. 11B). The hair 100 of the first internal magnet 64a and the hair 100 of the second internal magnet 64b are inclined in opposite directions with respect to the peripheral direction. Furthermore, the hair 100 has elasticity and is formed of, e.g., a wire. Each extended end of the hair 100 is frictionally engaged with an outer peripheral surface of a lens frame 61 which is mounted inside the inner main body portion 76.

This lens frame 61 is rotatable around the central axis and slidable in the axial direction with respect to the inner main body portion 76. However, a cam pin 67 is provided to protrude on the outer peripheral surface of the lens frame 61. This cam pin 67 is inserted into and engaged with a cam groove 70 piercing the inner main body portion 76. This cam groove 70 is spirally extended with respect to the central axis L.

A function of an endoscopic system 20 according to this embodiment will now be described. When performing focus adjustment, the first or second switch 94a or 94b is operated. When the first switch 94a is pressed, as indicated by an arrow B in FIG. 11C, the first external magnet 58a is moved toward the inner side in the radial direction. Moreover, a distance between the first external magnet 58a and the first internal magnet 64a is reduced, and a repulsive force between the first external magnet 58a and the first internal magnet 64a is increased. As a result, as shown in FIG. 11D, the first internal magnet 64a is moved toward the inner side in the radial direction, and the hair 100 is deflected in a V-like shape. The hair 100 is restored to a linear shape by its own elastic force, and the lens frame 61 is rotated around the central axis at this time. Since the cam pin 67 of the lens frame 61 is moved along the cam groove 70, the lens frame 61 is moved around the central axis and also moved in one axial direction. Then, when the pressed state of the first switch 94a is released, the first external magnet 58a is restored to its original state by the flexibility of the first tabular member 98a, and the first internal magnet 64a and its hair 100 are also restored to their original states. When such a pressing operation of the first switch 94a is repeated, the lens frame 61 is moved in one axial direction.

When the second switch 94b is pressed, similarly to the first switch 94a, the lens frame 61 is rotated around the central axis. However, since the hair 100 of the second internal magnet 64b is inclined in the direction opposite to that of the hair 100 of the first internal magnet 64a with respect to the peripheral direction, the lens frame 61 is rotated in the opposite direction and also moved in the opposite axial direction. The first switch 94a and the second switch 94b are operated in this manner to perform focus adjustment.

Therefore, an endoscopic system 20 according to this embodiment demonstrates the following effect. The first and second internal magnets 64a and 64b are reciprocated in the radial direction by reciprocating the first and second external magnets 58a and 58b in the radial direction with respect to the housing 56. Further, the lens frame 61 is reciprocated in the axial direction through the hair 100 by movement of each of the first and second internal magnets 64a and 64b in the radial direction. That is, the first and second external magnets 58a and 58b do not have to rotated around the central axis in order to reciprocate the lens frame 61 in the axial direction. Therefore, focus adjustment can be readily carried out even under the circumstances where the rotating operation is restricted.

FIG. 12 shows a modification of the seventh embodiment according to the present invention. Like reference numerals denote structures having the same functions as those of the seventh embodiment, thereby obviating a description thereof. A camera head according to this embodiment has a main body portion 35, first and second switches 94a and 94b, a housing 56, an inner main body portion 76 and first and second internal magnets 64a and 64b which are the same as those in the seventh embodiment. However, hair 100 of the first internal magnet 64a is inclined in one axial direction within a plane parallel to a plane including a central axis L of the housing 56 and a through axis of a first through hole 96a. Hair 100 of the second internal magnet 64b is inclined in the other axial direction within the same plane. Furthermore, a lens frame 61 is slidable in an axial direction, and does not have a cam pin 67 (see FIG. 11A) protruding thereon and a cam groove 70 (see FIG. 11A) formed on the inner main body portion 76.

A function and an effect of an endoscopic system 20 according to this embodiment will now be described. When the first switch 94a is pressed, the first internal magnet 64a is moved toward the inner side in the radial direction like the seventh embodiment. As a result, the lens frame 61 is moved in one axial direction by an operation of the hair 100 which is the same as that in the seventh embodiment. On the other hand, when the second switch 94b is pressed, the lens frame 61 is moved in an opposite axial direction. Therefore, this modification demonstrates the same function and effect as those of the seventh embodiment.

In the foregoing embodiments, the magnets may be arranged with south poles and north poles all being reversed, and an electromagnet may be used as each magnet. Moreover, the description has been given on the airtight container whose inside is airtightly held as an example of the sealed container in the foregoing embodiments. However, any container can be used as the sealed container as long as its inside is sealed, and a liquidtight container whose inside is liquidtightly held may be adopted. Additionally, although the description has been given as to the camera head of the endoscope as an example in the foregoing embodiments, the present invention can be applied to any medical apparatus which has a sealed container whose inside is sealed and in which various kinds of members in the sealed container is operated by utilizing a magnetic force between magnets arranged inside and outside the sealed container.

### Industrial Applicability

The present invention provides the medical apparatus which has a sealed container whose inside is sealed, in which various kinds of members in this sealed container is operated securely by utilizing a magnetic force between magnets arranged outside and inside the sealed container and whose operability is improved.

## Claims

1. Medical apparatus **characterized by** comprising:
a sealed container whose inside is sealed;
at least one external magnet which is provided outside the sealed container to be movable with respect to the sealed container;
an external member which is provided outside the sealed container and moves the external magnet;
at least one internal magnet which is provided in the sealed container to be movable with respect to the sealed container; and
an internal member which is provided in the sealed container and moved by movement of the internal magnet,
wherein the external magnet and the internal magnet are arranged in such a manner that the internal magnet is moved by the action of a repulsive force between the external magnet and the internal magnet based on movement of the external magnet.

2. The medical apparatus according to claim 1, **characterized in that** the plurality of external magnets is provided, reciprocable with respect to the sealed container and arranged side by side in a reciprocating direction, and
the internal magnet is arranged between the external magnets with respect to the reciprocating direction.

3. The medical apparatus according to claim 1, **characterized in that** the plurality of internal magnets is provided, reciprocable with respect to the sealed container and arranged side by side in a reciprocating direction, and
the external magnet is arranged between the internal magnets with respect to the reciprocating direction.

4. The medical apparatus according to any of claims 1 to 3, **characterized in that** the sealed container has a cylindrical housing,
the external member has an external annular member which is mounted outside the housing to be rotatable around a longitudinal axis of the housing,
the internal member has an internal annular member which is mounted inside the housing to be rotatable around the longitudinal axis of the housing, and
the external magnet is provided to the external annular member and the internal magnet is provided to the internal annular member.

5. The medical apparatus according to any of claims 1 to 3, **characterized in that** the sealed container has a cylindrical housing,
the external member has an external annular member which is mounted outside the housing to be movable in a longitudinal axial direction of the housing,
the internal member has an internal annular member which is mounted inside the housing to be movable in the longitudinal axial direction of the housing, and
the external magnet is provided to the external annular member and the internal magnet is provided to the internal annular member.

6. The medical apparatus according to claim 1, **characterized in that** the sealed container has an urging member, and
the internal member is movable in one direction by the internal magnet and movable in an opposite direction by urging of the urging member.

7. The medical apparatus according to claim 6, **characterized in that** the sealed container has a cylindrical housing,
the external magnet is movable in a longitudinal axial direction of the housing,
the internal member has an internal annular member which is mounted inside the housing to be movable in the longitudinal axial direction of the housing, and
the internal magnet is provided to the internal annular member.

8. The medical apparatus according to claim 1, **characterized in that** the sealed container has a cylindrical housing,
the external magnet is reciprocable in a radial direction of a longitudinal axis of the housing, and
the internal magnet is reciprocable in the radial direction.
